Europäisches Patentamt

European Patent Office

Office européen des brevets

(11) Publication number: **0 268 567**
**A2**

## (12) EUROPEAN PATENT APPLICATION

(21) Application number: 87850280.6

(22) Date of filing: 14.09.87

(51) Int. Cl.⁴: **A 61 B 19/08**

(30) Priority: 16.09.86 SE 8603902

(43) Date of publication of application:
25.05.88 Bulletin 88/21

(84) Designated Contracting States:
AT BE CH DE ES FR GB IT LI NL SE

(71) Applicant: Mölnlycke AB
S-405 03 Göteborg (SE)

(72) Inventor: Frisk, Johan
25, Av. de Beaulieu
B-1160 Bruxelles (BE)

Sallets, Ignace
25, Av. de Beaulieu
B-1160 Bruxelles (BE)

(74) Representative: Hammar, Ernst et al
H. ALBIHNS PATENTBYRA AB Box 7664
S-103 94 Stockholm (SE)

The title of the invention has been amended (Guidelines for Examination in the EPO, A-III, 7.3).

(54) Surgical drape for use when performing head surgery.

(57) The present invention relates to a surgical drape for use in head surgery, comprising a head sheet (1) which is intended to be wound around a patient's head in the manner of a turban, and a protective sheet (2) which has a greater length and area than the head sheet and which is intended to serve as an underlay for the head sheet, wherein the head sheet is placed on top of the protective sheet in the upper part of the sheet and the surgical drape comprised of these sheets is folded in a manner such that both sheets can be placed beneath the head of a patient in one working step. According to the invention the lower part of the protective sheet has formed therein a slit (3) which divides the strip into two symmetrical parts and which extends from the bottom edge (22) of the protective sheet up to at least the bottom edge (12) of the head sheet, wherein the lower slitted part of the protective sheet can be used to cover the chest region of a patient.

The invention provides a surgical drape which is inexpensive in manufacture and easy to manipulate. The surgical drape also obviates in many surgical cases the need to use additional, conventional surgical drapes.

FIG.1

EP 0 268 567 A2

**Description**

## A surgical drape for use when performing head surgery

The present invention relates to a surgical drape for use when performing head surgery.

In order to be able to achieve a sterile environment when performing a surgical operation on the head of a patient, e.g. an eye operation, ear operation or plastic surgery, it is important to ensure that bacteria-carrying particles in the patient's hair are unable to fall into the operative area. This is normally achieved by winding or wrapping hand towels or the like around the patient's head in a turban-like fashion and then holding the towels in position with the aid of clamps or like fasteners, or by taping a plurality of surgical drapes (at least three) securely to the patients head around the surgical area.

One such surgical drape, however, is known from US-A-4,457,026, this known drape comprising a head sheet which is secured to a protective sheet and avoids the drawbacks associated with the use of hand towels and separate cloths. The head sheet of this surgical drape has a relatively complicated configuration in its position of use and includes parts which are glued together. Manufacture of the known surgical drape is therefore effected in two stages, by first folding the head sheet and glueing the same so that it obtains the configuration desired, and then securing the head sheet to the protective sheet along a glue line. The surgical drape comprising the head sheet and the protective sheet is then folded to a suitable packet form. The protective sheet also includes other mutually glued parts.

The object of the invention is to provide a surgical drape of the aforesaid kind which is easy to manufacture and to manipulate or drape, and to form the protective sheet so that in addition to forming an underlying support for the head sheet it will also protect the shoulders and chest region of a patient.

This object is achieved with an inventive surgical drape having the characteristic features set forth in Claim 1.

Because, as a result of the slit, the protective sheet comprises two parts which extend free from the patient's head and neck, these parts can be placed over the patient's chest. In many cases this obviates the need for further protective sheets. Furthermore, the head sheet is not glued to the protective sheet, which means that a soft material can be chosen for the head sheet, since relative displacement or twisting of the sheets will not induce any tension or stresses in the head sheet. This will enhance patient comfort. Because, when applied, the head sheet and the protective sheet are held together solely by the particular manner in which the sheets are folded, it is possible to combine together head sheets and protective sheets of mutually different shapes and sizes which affords a highly flexible surgical drape construction. Finally, the drape of the present invention can be readily manipulated, or draped, since rearward folding of the protective sheet from its head-sheet holding position to its position of use is effected by an oblique fowards-outwards-upwards movement of the hands of a theatre assistant, from a hand position beneath the folded corner flaps of the concertina-like folds of the lower part of the protective sheet. Insertion and arrangement of the sheet in its position of use can thus be effected in one single continuous movement.

These and other features and advantages will become more apparent from the following, more detailed description of an embodiment of an inventive surgical drape intended for use on the head of a patient, made with reference to the accompanying drawings, in which

Figure 1 illustrates one embodiment of a surgical drape according to the invention for head surgery;

Figures 2a-2e illustrate the various stages of folding the surgical drape illustrated in Figure 1 to the insertion configuration of the drape;

Figure 3 illustrates the surgical drape of Figure 2e with the side parts thereof folded inwardly;

Figure 4 illustrates the step of upwardly folding the drape shown in Figure 3 to an insertion configuration;

Figures 5a-5f illustrate the various stages of draping a surgical drape according to the invention around a patient's head and over his chest; and

Figure 6 illustrates a variant of the inventive surgical drape provided with a longer head sheet, and shows the drape arranged on a patient.

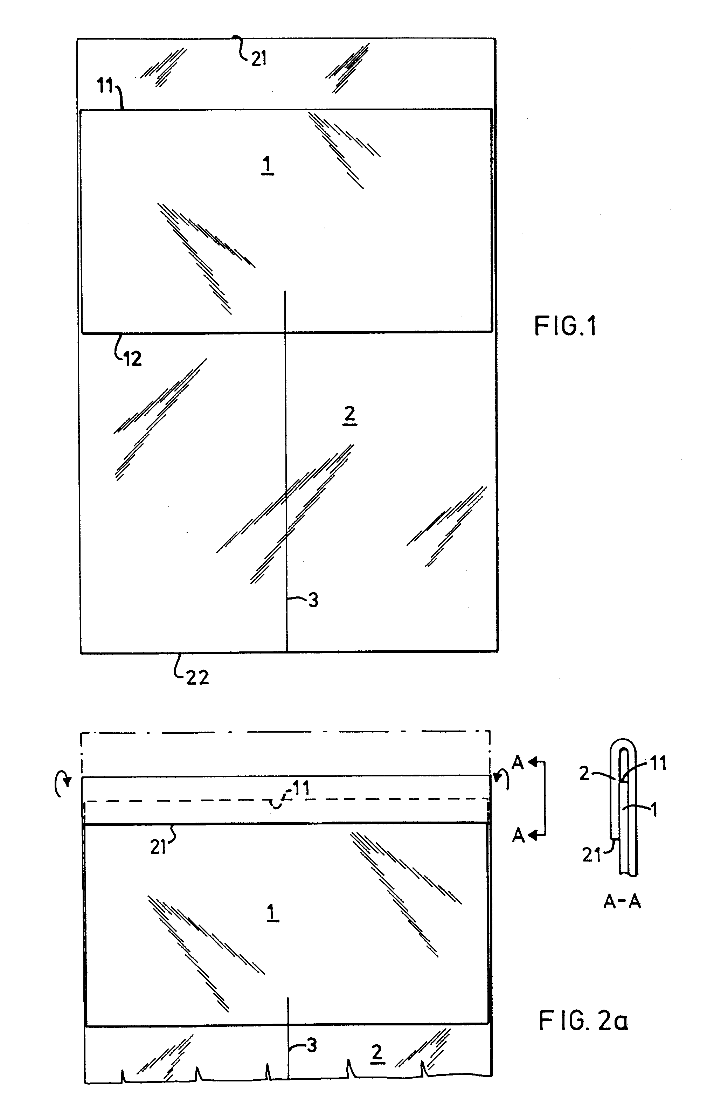

The surgical drape for use with head surgery illustrated in the drawings comprises two rectangular sheets, a head sheet 1 and a protective sheet 2, the head sheet being placed on the protective sheet prior to the folding the drape, as illustrated in Figure 1. The head sheet can be made from a soft material which will readily mould to the head contours and which can be readily draped, whereas the protective sheet can be made from a conventional surgical drape material. One example of suitable head-sheet material is so-called Sontara, which is spun-laced-fibre cloth manufactured and sold by E.I. Du Pont de Nemours and Company.

The sheets 1 and 2 may, of course, have a form different to that illustrated in Figure 1. For example, the protective sheet may have the shape of an iscoceles trapezoid, which further facilitates manipulation from the use configuration of the drape, as described hereinafter. Furthermore, the head sheet need not extend over the whole width of the protective sheet, even though this is preferred so as to ensure that the head sheet will always reach around the head of the patient. As illustrated in Figure 1, the relative dimensions of the sheets may also vary in the direction of their longitudinal extensions.

It will be seen from Figure 1 that the inventive surgical drape has formed therein an elongated slit 3, which divides the lower part of the protective sheet into two equal parts and which extends from the bottom edge 22 of the protective sheet to a point located somewhat inwardly of the head sheet 1. The slit 3 has two functions. It enables the lower part of the protective sheet to be used to cover the chest region of the patient and also facilitates manipulation of the drape when the drape is developed from its insertion configuration to its use configuration, as will be made more apparent in the following.

The prime function of the protective sheet 2 in other regards is to ensure the sterility of at least the rear side of the head sheet 1 resting loosely on the protective sheet, said rear side lying against the protective sheet until the head sheet is wrapped around the patient's head, whereupon the front side of the head sheet is brought into contact with the non-sterile hair of the patient while the rear side of said head sheet will be turned outwards and thus constitute the sterile part of the head sheet. It should be mentioned in this connection that the surgical drape must, of course, be capable of being draped without the theatre assistant touching the patient's hair.

Figures 2a-3e illustrate the steps taken when folding the head surgery drape from the configuration illustarted in Figure 1 to the drape inSertion configuration, i.e. the configuration in which the two sheets together can be pushed in beneath the head of a patient in one single movement, without touching the patient's hair. The contours of the drape prior to being folded in the manner intended are shown in chain lines in these Figures, whereas the arrows drawn in the Figures indicated the lines about which the folds are effected and in which direction. The Figures also show sectional side views of the folded drape.

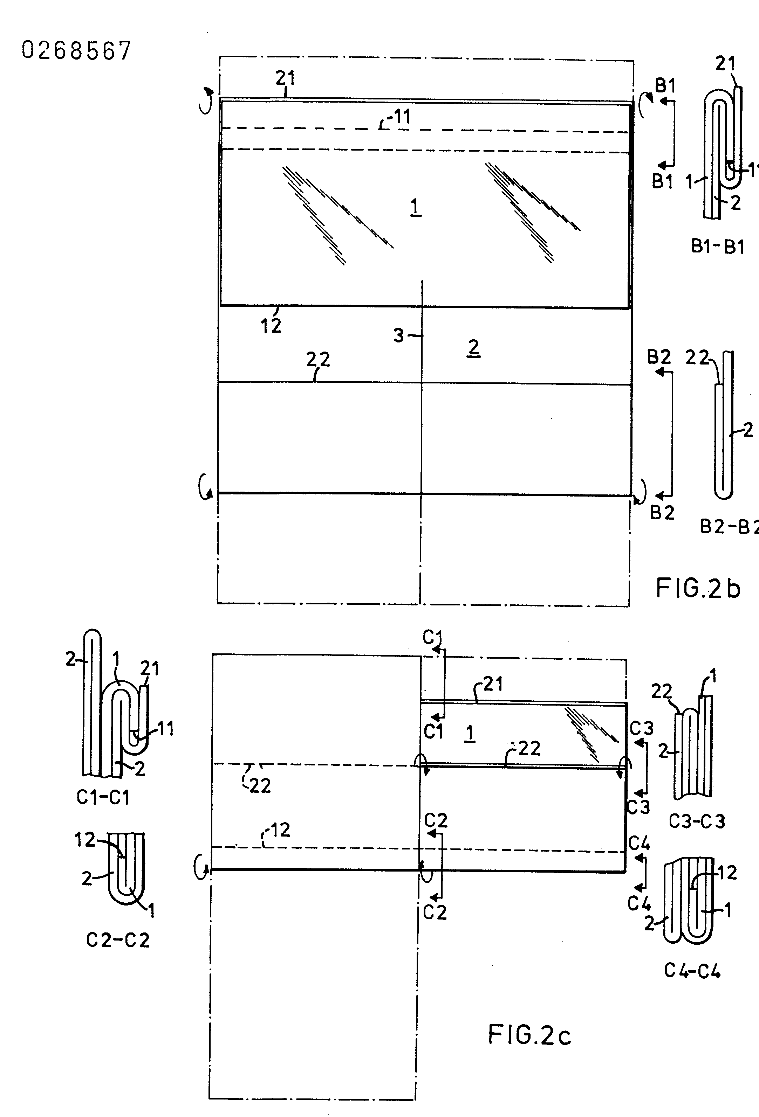

Thus it will be seen from Figures 2a and 2b that a portion of the upper part of the protective sheet 2 is folded-up over the upper portion of the head sheet 1 (see Figure 2a, in which the respective upper edge portions of the protective sheet and the head sheet are referenced 21 and 11), and that the upper drape portion formed by folding in this manner is then folded along a fold line which extends parallel with the upper edge 21 of the protective sheet subsequent to the first folding step, in under the surgical drape to the position illustrated in the upper part of Figure 2b. As will be seen from the side view B1-B1 shown in this Figure, the upper part of the surgical drape has a so-called concertina-fold, whereby the folded upper part of the surgical drape can be unfolded or extended, simply by pulling upwards on the upper edge portion 21 of the protective sheet. It is mentioned in this regard that the protective sheet either alone or together with the head sheet can be folded in a manner to produce a multiple of concertina-like folds, depending on the relative lengths of the sheets and on the desired length of the head sheet folded in said upper portion in relation to the length of the lower slitted part of the protective sheet.

It will be seen from the bottom part of Figure 2b that a portion of the lower part of the protective sheet is folded inwardly over the surgical drape along a transverse fold line which is located at a distance from the point at which the slit 3 terminates in the head sheet, this distance being substantially equal to two thirds of the total length of the slit.

As shown in Figure 2c, the thus folded lower portion of the protective sheet, together with the slitted portion of the head sheet, is folded in over the remaining part of the head sheet along a transverse line which extends through the end point of the slit 3 (see the left hand side of Figure 2c). A part of the lower portion of the protective sheet is then folded back along a fold line which is located at a distance from the end point of the slit, this distance being approximately equal to one third of the total length of the slit (see the right hand part of Figure 2c).

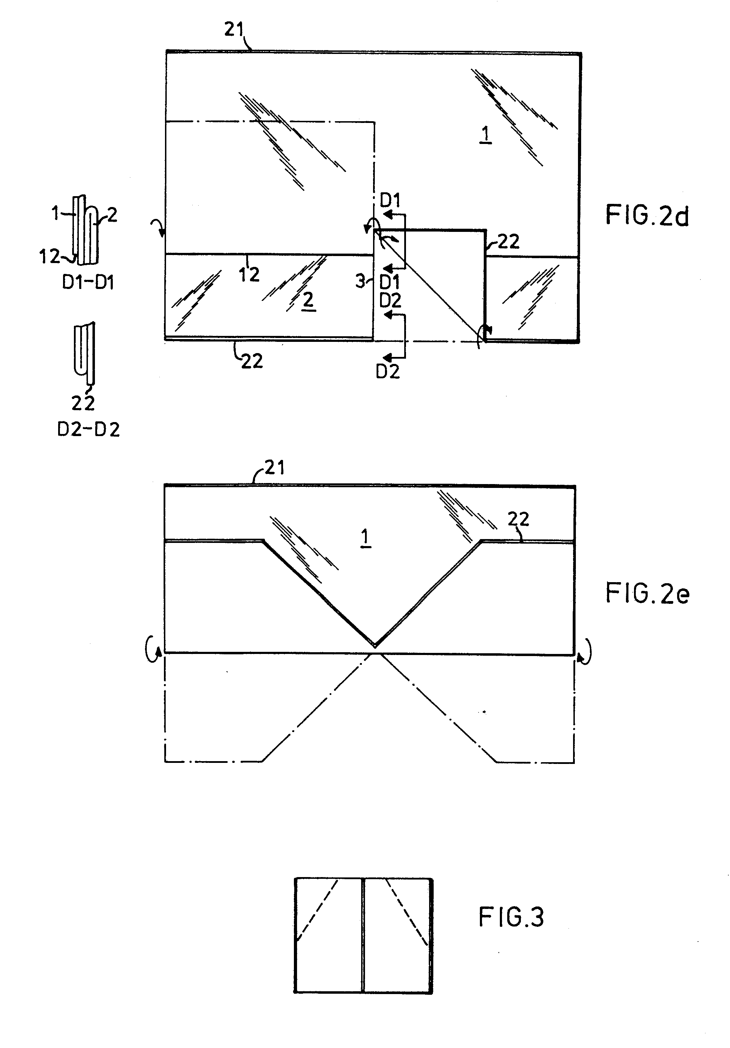

In the Figure 2d illustration the lower part of the protective sheet has been folded back along the transverse fold line extending through the end point of the slit (see the left hand part of the Figure). The side view D2-D2 shows that this lower part is folded in concertina fashion. Furthermore, the right hand part of the Figure shows that the corner flaps of the lower part of the protective sheet located nearest the slit are folded along a fold line which is inclined to the longitudinal extension. In this case the line should be chosen so that at least a part of the folded-over flap will cover the lowermost part of the head sheet. The lower part of the protective sheet with inwardly folded corner flaps is then folded in, back over the head sheet 1 along the fold line extending through the point at which the slit terminates. Subsequent to being folded in this manner, the surgical drape has the configuration illustrated in Figure 2e, which corresponds to the insertion configuration.

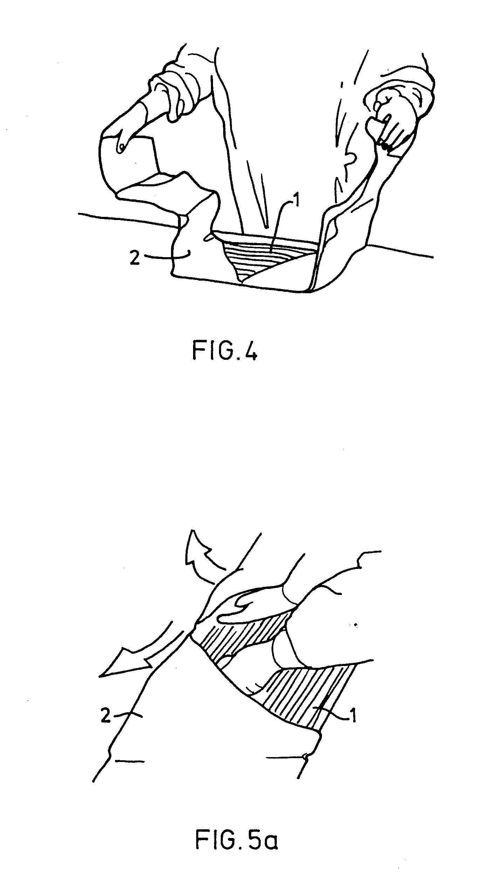

It should be mentioned here that the series of Figures 2a-2e solely illustrate one example of how the surgical drape can be folded in order to achieve the configuration illustrated in Figure 2e. It is, of course, obvious that the corner flaps can be folded-in from the configuration shown in the right hand part of Figure 2a, or with only a small backwards folding movement of the lower, slitted part of the protective drape. Furthermore, the concertina-like folds in the upper and lower parts of the surgical drape may be obtained in a manner different to that described. The series of Figures solely illustrate one method of folding the drape to the insertion configuration and are not therefore restrictive of the protective scope of the invention. Furthermore, it should be mentioned here that in practice the surgical drape is folded so that in the folded insertion configuration of the drape the upper and lower parts overlap one another (cf Figure 4), which in the Figure 1 configuration can be readily achieved, by choosing another fold line when folding the upper part of the drape in accordance with Figures 2a and 2b.

In Figure 3, the side parts have been folded in towards the centre of the head sheet with the aid of a concertina-like fold. From this configuration, one half of the surgical drape has been folded over the other half thereof, in order to obtain a folded drape package suitable for transportation. The thus folded drape is then placed in a suitable wrapper, to form a sterile transport package.

Figure 4 illustrates the unfolding or development of the side concertina-like folds of a transport-folded surgical drape, such as to bring the drape to its insertion configuration.

Figures 5a-5f illustrate the various steps taken when positioning an inventive surgical drape which has been folded to its insertion configuration.

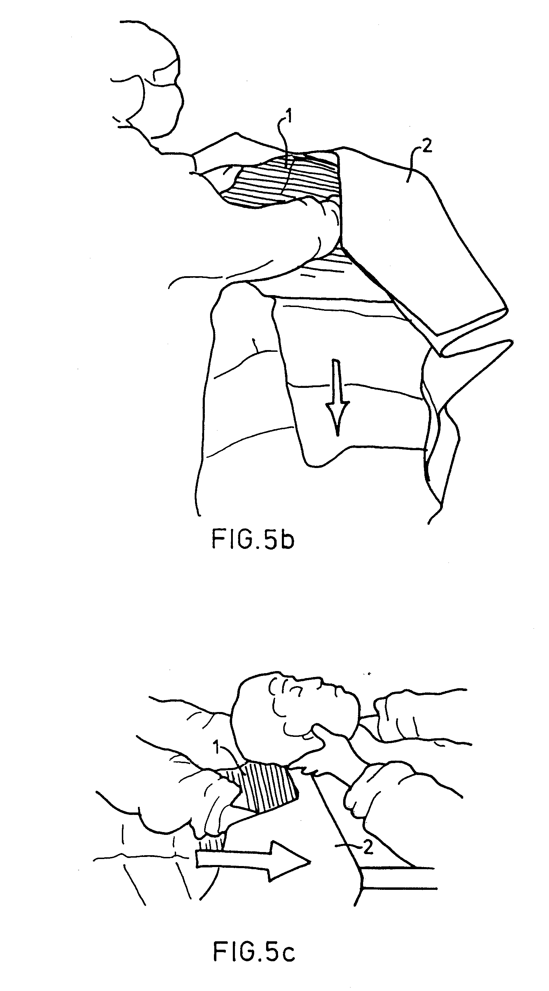

Figure 5a shows that a member of the theatre staff shall insert his hands beneath the inwardly folded corner flaps. The surgical drape is then lifted with the lower part of the head sheet facing upwards, as illustrated in Figure 5b, so that the folds of the concertina-folded upper part of the drape fall out naturally, i.e. gravitationally, as indicated by an arrow in Figure 5b. The inwardly folded corner flaps ensure herewith that the lower part of the surgical drape is not extended and consequently the head sheet is unable to fall down. The surgical drape is then placed on an operating table and inserted beneath the head of the patient, whilst the patient's head is held raised by another member of the theatre staff, as illustrated in Figure 5c. It should be mentioned that if some part of the drape comes into contact with the patient's hair when inserting the drape, which should normally not occur, this part of the drape will not face outwards when finally arranging the drape.

Subsequent to inserting the drape beneath the patient's head to the position desired, the patient's head is lowered onto the head sheet exposed by the presence of the inwardly folded corner flaps. The staff member then moves his hands, which are still inserted beneath the corner flaps, obliquely forwards-upwards-outwards, such as to fold back the lower part of the protective sheet to the position shown on the right of Figure 2. The corner flaps are then folded out, so that the lower part of the drape obtains the configuration shown in Figure 2d. The surgical drape is therewith in its position of use, which differs from the position illustrated on the left in Figure 2d solely by the fact that the upper part of the drape has been extended, or developed. In this configuration of the drape, the head sheet rests loosely on the protective sheet.

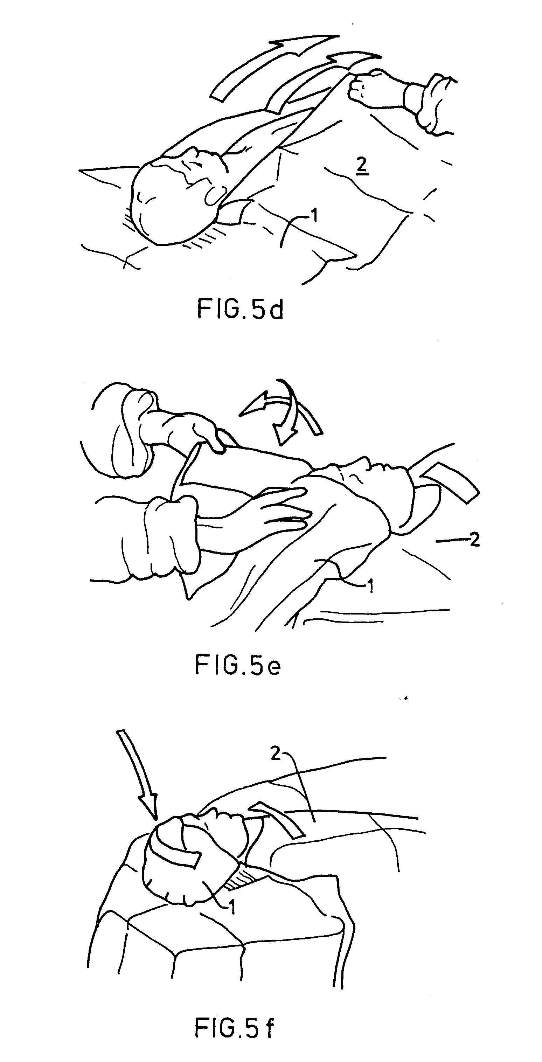

Manipulation of the surgical drape from its use configuration is preferably effected by first positioning the lower part of the protective sheet over the patient's chest region, by grasping the lower edge portion 22 of one half of the concertina-folded lower part of the protective sheet, and moving said edge portion downwards over the patient's chest, so as to unfold said lower part of the sheet, as shown in Figure 5d. The folds of the other lower, concertina-folded half of the protective sheet are then extended in a similar manner and said sheet half brought over the patient's chest region, whereafter the two sheet halves are taped together with adhesive tape.

Figures 5e and 5f illustrate how the head sheet can be wrapped or wound around the patient's head and then secured in its wrapped position by means of adhesive tape.

The adhesive tapes required to secure the sheet parts are preferably pre-packed in the transport package enclosing the surgical drape.

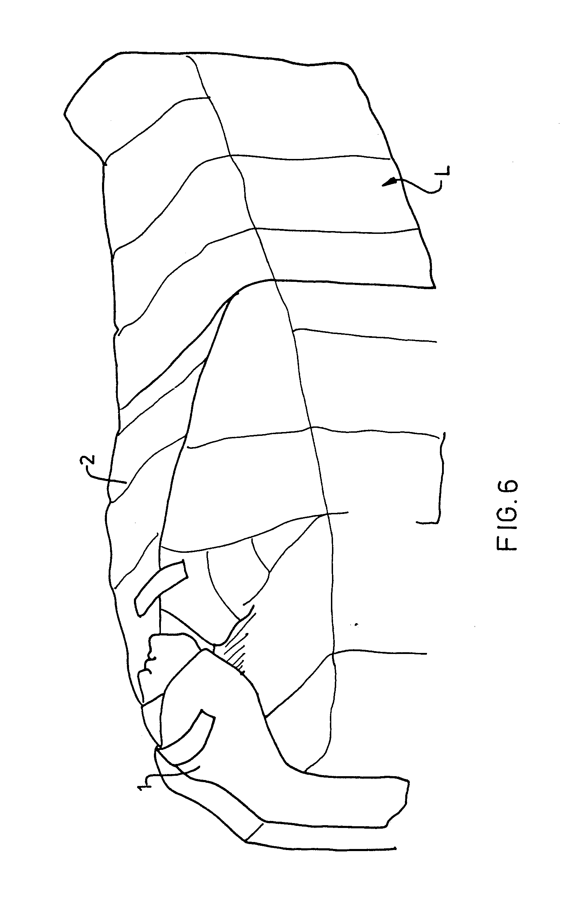

Figure 6 illustrates a surgical drape which incorporates a longer head sheet in the finally arranged state of the drape. This longer head sheet is intended to cover a breathing tube which extends towards the head end of the operating table. In this embodiment, the patient's trunk and legs are covered with a conventional surgical sheet L.

As beforementioned the protective sheet may have a trapezoidal shape. In this case the outermost ends of the bottom edge portion 22 of the lower slitted portion of the protective sheet will not be covered by overlying folded parts when the surgical drape is in its folded use configuration, so that these ends can be readily gripped.

Neither need the slit 3 extend into the head sheet. In this case, however, in order to prevent the head sheet from falling down when raising the drape (see Figure 5b), the line along which the drape is folded from its insertion configuration to its configuration to use must lie somewhat inwardly of the head sheet. The apex of the V formed by the inwardly folded corner flaps of the lower part of the protective sheet will be located at a distance from the bottom edge of the surgical drape in the folded insertion configuration of the drape.

Thus, the present invention provides a surgical drape for head surgery which is very simple to fabricate and the lower part of which will cover the chest region of a patient and therewith in the case of many operations obviate the need for additional conventional surgery drapes. Furthermore, the head sheet is freely moveable in relation to the protective sheet when the drape is extended to its configuration of use, thereby enabling the head sheet to be draped more readily than in the case of a drape where the head sheet is fastened to the protective sheet, and moreover can be made from a thinner or softer material, since unavoidable movements of the patient's head will not induce tensile stresses in the head sheet. This will enable the patient's head to be, e.g., twisted subsequent to positioning the head sheet, without the sheet tearing. Because of the particular way in which the drape is folded, there is formed a V-shaped recess in the lower part of the protective sheet which is folded in over the head sheet in the insertion configuration of the inventive surgical drape. This will ensure that a patient's head resting on the head sheet will not prevent the surgical drape from being extended from its insertion configuration to its configuration of use. The inwardly folded corner flaps also prevent the front side of the underportion of the protective sheet from coming into contact with the patient's hair during this downward folding movement. Furthermore, these inwardly folded corner flaps ensure that the lower part of the protective sheet will not be unintentionally unfolded, and the corner flaps also delimit well defined and readily accessible apertures into which the hand of a staff member can be inserted.

It will be evident from the aforegoing that the invention provides a well functioning and readily manipulated head surgical drape which comprises solely but a few parts and which can be manufactured inexpensively.

The described embodiment is only intended to provide an illustrative example which explains the invention in all its apsects and does not, of course, limit the scope of the inventive concept. The scope of the invention is therefore limited solely by the following claims.

## Claims

1. A surgical drape for use when performing head surgery, characterized by a head sheet (1) which is intended to be wrapped around a patient's head in a turban-like manner, and a protective sheet (2) which has a greater length and area than the head sheet and which is intended to form an underlay for the head sheet, wherein the head sheet is placed on top of the protective sheet in the upper part thereof and the surgical drape comprised of said sheets is folded in a manner such that both sheets can be placed beneath a patient's head in one working step; and in that the lower part of the protective sheet incorporates a slit (3) which divides sad lower part into two symmetrical portions and which extends from the bottom edge (22) of the protective sheet (1), therewith enabling the lower, slitted part of the protective sheet to be used to cover the shoulders and chest region of a patient.

2. A surgical drape according to Claim 1, characterized in that the slit (3) extends somewhat into the head sheet (1).

3. A surgical drape according to Claim 1, characterized in that the head sheet (1) is freely moveable relative to the protective sheet (2) when it has been moved to its position of use subsequent to inserting the surgical drape.

4. A surgical drape according to Claim 1, characterized in that in the insertion configuration of the surgical drape, i.e. the folded configuration of the drape when inserting said drape beneath the head of a patient, the lower, slitted part of the protective sheet (2) is folded in concertina-like fashion, such that its extension from the lower edge of the head sheet (1) in the insertion direction is smaller than or equal to the extension of the head sheet (1) in the opposite direction in this position of the drape; in that the corner parts of the concertina-like folded lower part of the protective sheet adjacent the slit are folded along a fold line which is inclined in relation to the insertion direction; and in that the concertina-like folded lower part of the protective sheet with the thus inwardly folded corner flaps is folded over the head sheet (1), wherewith the folded lower part of the protective sheet has a V-shaped configuration in the centre part of the head sheet, whereby the thus folded corner flaps of the lower part of the protective sheet under which the hands of a theatre staff member are placed when inserting the surgical drape beneath the head of a patient prevent the lower, slitted and concertina-like folded part of the protective sheet from being developed unintentionally when manipulating said drape.

5. A surgical drape according to Claim 4, characterized in that in the insertion configuration of the drape the head sheet (1) is folded back upon itself at its leading edge in the insertion direction, to prevent the head sheet from falling down when manipulating the surgical drape.

6. A surgical drape according to Claim 1, characterized in that the head sheet (1) has a rectangular shape and the protective sheet (2) has the shape of an iscoceles trapezoid.

0268567

FIG.1

FIG.2a

A-A

0268567

FIG.2b

B1-B1

B2-B2

FIG.2c

C1-C1

C2-C2

C3-C3

C4-C4

0268567

FIG.2d

FIG.2e

FIG.3

0268567

FIG.4

FIG.5a

0268567

FIG.5b

FIG.5c

0268567

FIG.5d

FIG.5e

FIG.5 f

0268567

FIG.6